# EUROPEAN PATENT SPECIFICATION

(11) **EP 3 777 731 B1**
(45) Date of publication and mention of the grant of the patent: **01.11.2023**
(21) Application number: 19795918.2
(22) Date of filing: 03.05.2019
(51) Int. Cl.: A61B 17/425, A61B 17/43, A61D 19/04, C12M 3/00, C12M 1/00

(54) **OOCYTE-HOLDING PIPETTE FOR SPERM INJECTION METHODS WITHOUT CYTOPLASMIC ASPIRATION**
OOZYTENHALT-PIPETTE FÜR SPERMAINJEKTIONSVERFAHREN OHNE ZYTOPLASMATISCHE ASPIRATION
PIPETTE DE MAINTIEN D'OVOCYTES UTILISÉE DANS DES PROCÉDÉS D'INJECTION SPERMATIQUE SANS ASPIRATION CYTOPLASMIQUE

(30) Priority: 03.05.2018 ES 201830624 U
(43) Date of publication of application: 17.02.2021
(73) Proprietor: Vergara Alcaide, Francisco, 18150 Gójar (Granada) (ES)
(72) Inventor: Vergara Alcaide, Francisco, 18150 Gójar (Granada) (ES)
(74) Representative: Sahuquillo Huerta, Jesús
(86) International application number: PCT/ES2019/070290
(87) International publication number: WO 2019/211503

(56) References cited:
- WO-A1-02/102257
- WO-A1-2009/088384
- WO-A1-2012/037642
- WO-A2-2010/056755
- CN-A- 101 125 105
- CN-A- 107 649 226
- DE-A1-102010 040 681
- JP-A- S63 222 680
- LYU Q F ET AL: "New technique for mouse oocyte injection via a modified holding pipette", REPRODUCTIVE BIOMEDICINE ONLINE, ELSEVIER, AMSTERDAM, NL, vol. 21, no. 5, 1 November 2010 (2010-11-01), pages 663-666, XP027451651, ISSN: 1472-6483, DOI: 10.1016/J.RBMO.2010.07.004 [retrieved on 2010-10-28]

## Description

### Object of the Invention

As expressed in the title of the present specification, the invention relates to an oocyte-holding pipette for sperm injection methods without cytoplasmic aspiration, providing the intended function thereof with advantages and novelty features that are described in detail below and constitute an alternative improvement in the current state of the art.

More specifically, the object of the invention focuses on a micropipette for use as means for holding an oocyte in intracytoplasmic sperm injection or ICSI methods, the novel structural configuration of which, unlike conventional pipettes, eschews the step of aspirating the cytoplasm with the injection needle to ensure the rupture of the cytoplasmic membrane, since the new pipette, provided with a flared end, holds the oocyte by suction, slightly deforming it inside said end, and said deformation causes an increase in oocyte turgor which allows breaking the cytoplasmic membrane with the injection pipette without first suctioning the cytoplasm.

### Field of Application of the Invention

The field of application of the present invention is comprised within the sector of the industry dedicated to the manufacture of medical instruments, focusing particularly on the field of microdevices applicable for cellular-level procedures and interventions, and more specifically to devices intended for use in assisted reproduction procedures.

### Background of the Invention

As is known, intracytoplasmic sperm injection or ICSI is an assisted reproduction technique consisting of the fertilization of oocytes by means of injecting a sperm into their cytoplasm using a micropipette, after obtaining and preparing the gametes for the purpose of obtaining embryos that can be transferred to the mother's womb.

To carry out said technique, the previously denuded oocyte must be held with a holding pipette, while the sperm is injected with another pipette, in this case an injection pipette.

The problem is that the membrane surrounding the oocyte is a hard-to-break membrane due to its high elasticity; in fact, the injection needle can be inserted into the cytoplasm without actually breaking the membrane (the membrane was microscopically invaginated by the injection pipette but did not break). The conventional way for breaking the membrane is to perform aspiration with the injection pipette until breaking the membrane. However, this leads to a disorganized cytoplasm that may critically affect the mitotic spindle (the location of which could only be estimated as it is not observed with routine ICSI technique) if its arrangement is altered during cytoplasmic aspiration.

The membrane must break for the proper release of the sperm into the cytoplasm, and also for the activation of the Ca++ or calcium ion channel mechanisms, which in turn intervene in the activation of cell division mechanisms.

To solve this problem, the applicant himself is the proprietor of utility model ES1187335U which describes a "pipette for retaining oocytes in intracytoplasmic sperm injection methods" the features of which allow holding the oocyte and performing injection through its membrane without prior cytoplasmic aspiration. Specifically, the configuration of said holding pipette has an innovative working end holding the oocyte by means of pressure, for which it has a decreasing frustoconical portion where the diameter of its cylindrical body is significantly reduced until defining a small support tip with a planar, straight, and angled configuration with respect to the cylindrical body.

Said holding pipette therefore holds the oocyte by means of a slight pressure, not by means of suction like conventional pipettes, which pressure causes an increase in oocyte turgor and thereby allows breaking the cytoplasmic membrane without having to aspirate the cytoplasm.

The objective of the present invention is to develop another different, alternative holding pipette model that does not require aspirating the cytoplasm with the injection pipette either, which model, unlike the one mentioned above, provides greater assurance when holding the oocyte, as this is an aspect of the pipette susceptible to improvement. Specifically, instead of acting by means of the working tip pressing on the oocyte, which may constitute a highly skilled operation so that the oocyte does not slide to either side when being pressed, the present invention acts by means of suctioning the oocyte with the holding pipette itself, just like conventional pipettes with the only difference being that while those (conventional) pipettes only perform aspiration to hold the oocyte without deforming it, this pipette (the pipette of the present invention) aspirates the oocyte to deform it while it is partially housed in the flared working end thereof, as will be explained in detail below.

Moreover, and as reference to the current state of the art, it must be indicated that the applicant at least is unaware of the existence of any other pipette with the same application for holding oocytes in intracytoplasmic sperm injection methods or another similar pipette having technical, structural, and constitutive features that are identical or like those of the pipette herein claimed.

In the document of QF LYU et al ["New technique for mouse oocyte injection via a modified holding pipette" Reproductive Biomedicine Online, Elsevier, Amsterdam (NL), vol.21, n°5, 1 November 2010, pages 663-666, ISSN: 1472-6483] it is disclosed a technique to improve mouse oocyte survival from intracytoplasmic, using a modified holding pipette with a trumpet-shaped opening.

In DE102010040681A1 it is disclosed a tool for automated gripping of active component beads for e.g. releasing human body, has pressure change device formed for changing pressure of work fluid to ambient pressure, and contact surface concavely formed relative to reference axes. The tool has a work fluid channel led into a bead contact surface formed at the tool. The work fluid channel is coupled or decoupled with a pressure change device at a coupling longitudinal end. The pressure change device is formed for changing pressure of a work fluid in the work fluid channel to ambient pressure. The bead contact surface is concavely formed relative to reference axes orthogonal to each other. The contact surface is made from sintered material. The beads comprise a support material i.e., bio polymer, such as agarose, and a biological active material.

Document CN101125105A relates to a micro-ovum holding pipette and the production method, which is used for the micro-operation on an ovum or embryo. The ovum or embryo which is held by the traditional micro-ovum holding pipettes is easy to slip or deform when the zona pellucida is punctured from the side surface. The end surface of an opening end of the novel micro-ovum holding pipette is processed into a rough surface, the roughening of the end surface of the opening end is arranged on the end surface by the melt adhesion of an electrothermal body under a microscope and is broken after cooling, so part surface of the smooth glass surface is irregularly adhered and removed, thus forming the rough end surface of the opening end. The front end of the micro-ovum holding pipette can be made into a larger pipe diameter, which is close to the diameter of the ovum or embryo of the animals (including the people).

Document JPS63222680 A discloses a micromanipulation injection needle device consisting of a very small injection needle inserted and fixed in the internal space of a very small pipette for suction and fixation of material to be treated, e.g. a cell. The end of the needle is positioned near the end of the pipette almost coaxially. The bases of the needle and the pipette are connected to separate pressure sources. The pressure source is, e.g. syringe or injector, and is connected to the needle or pipette through a flexible tube and nipple and seals of silicon rubber. The pressure applied to the inside of the pipette is negative (-1 kg/cm2) or active (1 kg/cm2).

The present invention can ensure the held ovum or embryo not to slip easily when the side surface carries out the micro puncture and the zona pellucida can be

punctured easily, thus improving the operation efficiency and reducing the deformation of the ovum or embryo in extrusion.

### Brief Description of the Invention

The invention is defined by appended claim 1. The oocyte-holding pipette for sperm injection methods without cytoplasmic aspiration proposed by the invention is therefore configured as a remarkable novelty in its field of application, given that the objectives indicated above are specifically and satisfactorily achieved in view of its implementation, with the characterizing details making it possible and distinguishing same being suitably described in the final claims accompanying the present description.

Specifically, as described above, the invention proposes a pipette for holding oocytes in intracytoplasmic sperm injection methods which fulfills said purpose of holding without cytoplasmic aspiration, as it eschews such step of performing aspiration with the injection needle, which is required in conventional holding pipettes to ensure the rupture of the cytoplasmic membrane and cause the activation of the oocyte and to more readily deposit the sperm in the cytoplasm, but which, as pointed out in the preceding section, may damage the original structure of the cytoplasm and, in some cases, alter the structure of the mitotic spindle.

To that end, the holding pipette of the present invention comprises an elongate and narrow cylindrical body, the working end of which, i.e., the end used for holding the oocyte, is characterized by having a flared funnel-shaped configuration communicating through its smaller-diameter end with the aspiration conduit running along the cylindrical body, the larger opposite end being sized to receive the oocyte in a tight-fitting manner, such that, upon aspirating said oocyte, it remains trapped inside said flared end and becomes deformed enough so as to have a more turgid surface which facilitates the application of the injection pipette without having to previously aspirate the cytoplasm with the pipette.

Therefore, as with the conventional holding pipette, the pipette without cytoplasmic aspiration of the invention fulfills the purpose of holding the oocyte in ICSI methods but eschews the step of aspirating the cytoplasm with the injection needle, which is required in conventional holding pipettes having a rounded working end to ensure the rupture of the cytoplasmic membrane and cause the activation of the oocyte and to more readily deposit the sperm in the cytoplasm, but which damages the original structure of the cytoplasm and, in some cases, may alter the structure of the mitotic spindle.

Similarly to conventional holding pipettes, the holding pipette of the invention is manufactured from embryo-tested glass, specifically borosilicate, and the method for manufacturing the holding pipette of the invention is also similar to the method for manufacturing these conventional holding pipettes.

In turn, the dimensions of the pipette and its parts are conceived so that it can be used in the same microinjection equipment in which conventional holding pipettes are used, only slightly changing the ICSI technique protocol.

The described pipette therefore represents an innovation in structural and constitutive features that have been unknown up until now, and these reasons, combined with its practical usefulness, provide it with sufficient grounds to obtain the exclusive privilege that is sought.

### Description of the Drawings

To complement the description that is being made and for the purpose of helping to better understand the features of the invention, a set of drawings is attached to the present specification as an integral part thereof in which the following is depicted in a non-limiting, illustrative manner:
Figure 1 shows a side elevational view of the holding pipette object of the invention, with its general configuration and the main parts it comprises being observed.
Figure 2 shows an enlarged view of detail A indicated in Figure 1 and corresponding to the working end of the pipette, according to the invention, with the general configuration thereof being observed.
Figures 3 and 4 show respective enlarged views according to a longitudinal section view and an enlarged front elevational view, respectively, of the working end of the pipette shown in Figure 2, with the configuration and dimensions thereof being observed in greater detail.
Figures 5 and 6 show respective schematic views of the mode of operation of the working end of the pipette of the invention for holding the oocyte by means of suction and deformation of the oocyte and without aspiration of its cytoplasm.

### Preferred Embodiment of the Invention

In view of the mentioned figures and according to the numbering used, a non-limiting embodiment of the holding pipette of the invention can be seen therein, which pipette comprises the parts and elements indicated and described in detail below.

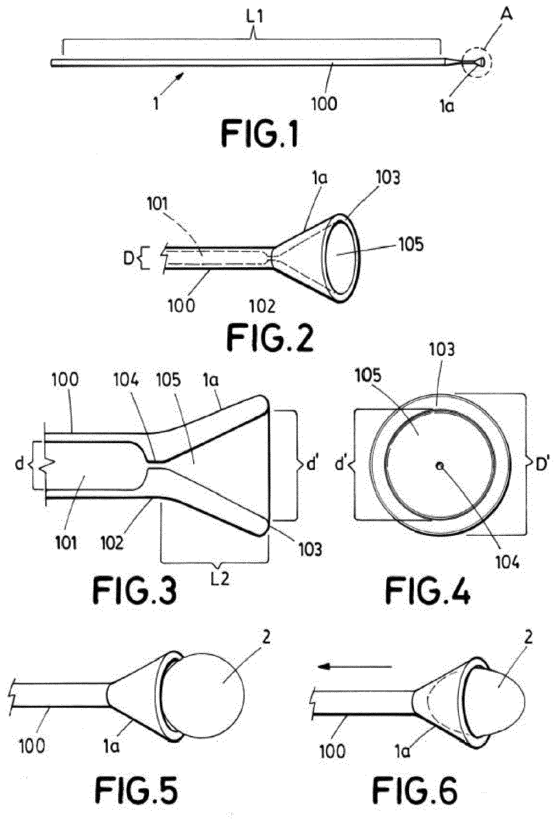

In this sense, as seen in said figures, the holding pipette (1) of the invention comprises an elongate, narrow, and hollow cylindrical body (100), inside which runs a suction conduit (101) that communicates with a working end (1a) for holding the oocyte (2), which end is characterized by having a flared funnel-shaped configuration defining an internal conical cavity (105), the smaller-diameter proximal end (102) of which communicates with the mentioned suction conduit (101), the larger-diameter distal end (103) being sized to receive the oocyte (2) in a tight-fitting manner, such that, upon aspirating same via suction conduit (101), the oocyte remains partially trapped inside and becomes deformed, as shown in Figures 5 and 6, changing from its spherical shape in the resting state to an oval shape that tensions the surface and determines a more turgid oocyte which facilitates the application of the injection pipette without having to previously aspirate the cytoplasm with the pipette.

In accordance with the invention, the cylindrical body (100) has a length (L1) of about 5 cm and an external diameter (D) of 500 micra, and the suction conduit (101) has a constant internal diameter (d) of 80 micra along its entire length with the exception of the point at which it is joined to the funnel-shaped working end (1a), there being, in the outlet opening of said conduit (101), coinciding with the smaller-diameter proximal end (102) of said funnel, a constriction (104) where said diameter is reduced considerably to preferably only about 17 micra.

Furthermore, the internal conical cavity (105) defining said funnel-shaped working end (1a) also preferably has a length (L2) of 128 micra and an internal diameter (d), at its larger-diameter distal end (103), of 120 micra, whereas the external diameter (D) of said distal end (103) of the working end (1a) is preferably 180 micra.

Having sufficiently described the nature of the present invention, as well as the manner of putting it into practice, it is not considered necessary to expand on this explanation so that one skilled in the art will understand its scope and the advantages derived from it, hereby stating that within its essential nature, the invention may be carried out to practice in other embodiments which differ in detail from that indicated by way of example and which will likewise attain the protection that is sought provided that its fundamental principle is not altered, changed, or modified.

## Claims

1. An oocyte-holding pipette for sperm injection methods without cytoplasmic aspiration comprising an elongate and hollow cylindrical body (100), inside which runs a suction conduit (101) that communicates with a working end (1a) for holding an oocyte (2); said working end (1a) has a flared funnel-shaped configuration defining an internal conical cavity (105), a smaller-diameter proximal end (102) of which communicates with the suction conduit (101) and a larger-diameter distal end (103) of which is sized to receive the oocyte (2) in a tight-fitting manner, such that, upon aspirating the oocyte (2) via the suction conduit (101), the oocyte (2) remains partially trapped inside and becomes deformed, changing from its spherical shape in a resting state to an oval shape that tensions the surface and increases the turgor of the oocyte which is arranged for the application of an injection pipette without having to previously aspirate the cytoplasm with the injection pipette;
**characterised in that**
the cylindrical body (100) has a length (L1) of 5 cm and an external diameter (D) of 500 micra, and the suction conduit (101) has a constant internal diameter (d) of 80 micra along its entire length with the exception of a point at which it is joined to the funnel-shaped working end (1a), there being, in an outlet opening of said conduit (101), coinciding with the smaller-diameter proximal end (102) of said working end (1a), a constriction (104) where the internal diameter of the suction conduit is reduced considerably.

2. The oocyte-holding pipette for sperm injection methods without cytoplasmic aspiration according to claim 1, wherein the constriction (104) the diameter of the conduit (101) is reduced to only 17 micra.

3. The oocyte-holding pipette for sperm injection methods without cytoplasmic aspiration according to any of claims 1 to 2, wherein the internal conical cavity (105) defining the funnel-shaped working end (1a) has a length (L2) of 128 micra and an internal diameter (d'), at its larger-diameter distal end (103), of 120 micra.

4. The oocyte-holding pipette for sperm injection methods without cytoplasmic aspiration according to claim 3, wherein an external diameter (D') of the distal end (103) of the funnel-shaped working end (1a) is 180 micra.

## Patentansprüche

1. Eine Eizellen haltende Pipette für Spermieninjektionsmethoden ohne zytoplasmatische Aspiration, umfassend einen länglichen und hohlen zylindrischen Körper (100), in dessen Inneren eine Saugleitung (101) verläuft, die mit einem Arbeitsende (1a) zum Halten einer Eizelle (2) in Verbindung steht. ; das Arbeitsende (1a) hat eine aufgeweitete trichterförmige Konfiguration, die einen inneren konischen Hohlraum (105) definiert, dessen proximales Ende (102) mit kleinerem Durchmesser mit der Saugleitung (101) kommuniziert und dessen distales Ende (103) mit größerem Durchmesser kommuniziert ), dessen Größe so bemessen ist, dass es die Eizelle (2) eng anliegend aufnimmt, sodass die Eizelle (2) beim Absaugen der Eizelle (2) über den Saugkanal (101) teilweise im Inneren eingeschlossen bleibt und sich verformt, Veränderung von der Kugelform im Ruhezustand zu einer ovalen Form, die die Oberfläche spannt und den Turgor der Eizelle erhöht, die für die Anwendung einer Injektionspipette geeignet ist, ohne dass zuvor das Zytoplasma mit der Injektionspipette abgesaugt werden muss;
**dadurch gekennzeichnet**
der zylindrische Körper (100) hat eine Länge (L1) von 5 cm und einen Außendurchmesser (D) von 500 Mikrometern, und die Saugleitung (101) hat über ihre gesamte Länge einen konstanten Innendurchmesser (d) von 80 Mikrometern mit Ausnahme einer Stelle, an der es mit dem trichterförmigen Arbeitsende (1a) verbunden ist, wobei es sich in einer Auslassöffnung der Leitung (101) befindet, die mit dem proximalen Ende (102) kleineren Durchmessers des trichterförmigen Arbeitsendes zusammenfällt (1a) eine Verengung (104), bei der der Innendurchmesser des Saugkanals erheblich verringert ist.

2. Die Eizellen haltende Pipette für Spermieninjektionsverfahren ohne zytoplasmatische Aspiration nach Anspruch 1, wobei die Verengung (104) den Durchmesser der Leitung (101) auf nur 17 Mikrometer reduziert.

3. Die Eizellen haltende Pipette für Spermieninjektionsverfahren ohne zytoplasmatische Aspiration nach einem der Ansprüche 1 bis 2, wobei der innere konische Hohlraum (105), der das trichterförmige Arbeitsende (1a) definiert, eine Länge (L2) von 128 Mikrometern aufweist und einen Innendurchmesser (d') an seinem distalen Ende (103) mit größerem Durchmesser von 120 Mikrometern.

4. Die Eizellen haltende Pipette für Spermieninjektionsverfahren ohne Zytoplasmatische Aspiration nach Anspruch 3, **dadurch gekennzeichnet, dass** ein Außendurchmesser (D') des distalen Endes (103) des trichterförmigen Arbeitsendes (1a) 180 Mikrometer beträgt.

## Revendications

1. Pipette porte-ovules pour méthodes d'injection de sperme sans aspiration cytoplasmique comprenant un corps cylindrique allongé et creux (100), à l'intérieur duquel passe un conduit d'aspiration (101) qui communique avec une extrémité de travail (1a) pour contenir un ovocyte (2). ; ladite extrémité de travail (1a) a une configuration en forme d'entonnoir évasé définissant une cavité conique interne (105), dont une extrémité proximale de plus petit diamètre (102) communique avec le conduit d'aspiration (101) et une extrémité distale de plus grand diamètre (103 ) dont est dimensionné pour recevoir l'ovocyte (2) de manière étanche, de telle sorte que, lors de l'aspiration de l'ovocyte (2) via le conduit d'aspiration (101), l'ovocyte (2) reste partiellement emprisonné à l'intérieur et se déforme, passer de sa forme sphérique au repos à une forme ovale qui tend la surface et augmente la turgescence de l'ovocyte qui est agencé pour l'application d'une pipette d'injection sans avoir à aspirer au préalable le cytoplasme avec la pipette d'injection ;
**caractérisé en ce que**
le corps cylindrique (100) a une longueur (L1) de 5 cm et un diamètre externe (D) de 500 microns, et le conduit d'aspiration (101) a un diamètre interne constant (d) de 80 microns sur toute sa longueur avec le à l'exception d'un point où il est relié à l'extrémité de travail en forme d'entonnoir (1a), dans une ouverture de sortie dudit conduit (101) coïncidant avec l'extrémité proximale de plus petit diamètre (102) de ladite extrémité de travail en entonnoir ( 1a), un rétrécissement (104) où le diamètre interne du conduit d'aspiration est considérablement réduit.

2. Pipette porte-ovocytes pour les méthodes d'injection de sperme sans aspiration cytoplasmique selon la revendication 1, dans laquelle l'étranglement (104) du diamètre du conduit (101) est réduit à seulement 17 microns.

3. Pipette porte-ovocytes pour les méthodes d'injection de sperme sans aspiration cytoplasmique selon l'une quelconque des revendications 1 à 2, dans laquelle la cavité conique interne (105) définissant l'extrémité de travail en forme d'entonnoir (1a) a une longueur (L2) de 128 microns. et un diamètre interne (d'), au niveau de son extrémité distale de plus grand diamètre (103), de 120 microns.

4. Pipette porte-ovocytes pour les méthodes d'injection de sperme sans aspiration cytoplasmique selon la revendication 3, **caractérisée en ce qu'**elle est **caractérisée en ce que** le diamètre externe (D') de l'extrémité distale (103) de l'extrémité de travail en forme d'entonnoir (1a) est de 180 microns.
